(19) Europäisches Patentamt
European Patent Office
Office européen des brevets

(11) Publication number: **0 178 668**
**A2**

(12) **EUROPEAN PATENT APPLICATION**

(21) Application number: 85113204.3

(22) Date of filing: 17.10.85

(51) Int. Cl.[4]: C 07 C 87/40
C 07 C 87/00

(30) Priority: 19.10.84 US 663663

(43) Date of publication of application:
23.04.86 Bulletin 86/17

(84) Designated Contracting States:
AT BE CH DE FR GB IT LI LU NL SE

(71) Applicant: E.I. DU PONT DE NEMOURS AND COMPANY
1007 Market Street
Wilmington Delaware 19898(US)

(72) Inventor: Liu, John Jih-Hua
204 Cain Rue
Newark Delaware 19711(US)

(74) Representative: von Kreisler, Alek, Dipl.-Chem. et al,
Deichmannhaus am Hauptbahnhof
D-5000 Köln 1(DE)

(54) **Process for preparing rimantadine.**

(57) A process for preparing rimantadine from 1-adamantanecarbonitrile is provided. The process comprises contacting 1-adamantanecarbonitrile in an ether solvent with $CH_3M$ where M is lithium or MgX wherein X is Br or Cl, and contacting the intermediate compound with a reducing agent, such as lithium aluminum hydride or catalytic hydrogenation, to form rimantadine free base. Salts can be formed from the free base, preferably the hydrochloride salt by reaction with HCl.

EP 0 178 668 A2

Title BP-6268

# PROCESS FOR PREPARING RIMANTADINE

## Background of the Invention

### Field of Invention:

This invention relates to processes for preparing rimantadine and more particularly to such processes from 1-adamantanecarbonitrile.

### Prior Art:

Pharmaceutical compositions containing α-methyl-1-adamantanemethylamine or the hydrochloride salt thereof (both herein referred to as rimantadine) are useful antiviral agents. Rimantadine and related compounds useful as antivirals were first described by Pritchard in U.S. Patents 3,352,912 and 3,592,934. Both patents describe the preparation of rimantadine from the corresponding ketone oxime by reduction with lithium aluminum hydride. This preparation is also described in Aldrich et al., J. Med. Chem., 14, 535 (1971).

Brake in U.S. Patent 3,489,802 describes the preparation of rimantadine by the reductive amination of the corresponding acetyl compound. In this process, the acetyl compound, hydrogen, ammonia and catalyst (cobalt, ruthenium, or nickel) are reacted at temperatures up to 250°C, e.g., 140-250°C, and pressures up to 15,000 psi, e.g., 500-2000 psi. This process on a commercial scale would require expensive, special reductive amination equipment.

Another rimantadine preparation process is described by Polis and Grava in U.S. Patent 3,852,352. This is a Leuckart-Wallach reaction in which rimantadine is prepared by reacting 1-adamantyl methyl ketone with ammonium formate, formamide, or a mixture of formamide or acetamide with formic acid.

Co-pending USSN 613,374, filed May 23, 1984 discloses the preparation of rimantadine by the reduction of 1-adamantyl methyl ketoxime by contacting a solution of the ketoxime with hydrogen in the presence of a platinum on carbon catalyst at a temperature in the range of about 10-60°C and a pressure of about 25 to 215 psia.

VanLeusen et al., Syn. Comm., 8, 397-401 (1978), disclose the preparation of 2-acyladamantanes by the reaction of 2-cyanoadamantane with alkyl lithiums followed by acidic hydrolysis to the ketones.

Pohland et al., J. Am. Chem. Soc., 75, 5898-5899 (1953), disclose the reduction of Grignard-nitrile adducts to primary amines using lithium aluminum hydride.

## Summary of the Invention

According to the present invention there is provided a process for the preparation of rimantadine comprising: contacting 1-adamantanecarbonitrile in an ether solvent with $CH_3M$ where M is lithium or MgX wherein X is Br or Cl, and contacting the intermediate compound with a reducing agent to form rimantadine free base.

As used herein psia = pounds per square inch absolute pressure and KPa = pressure in kilopascals.

## Detailed Description of the Invention

The process of the present invention provides a convenient process for preparing rimantadine in high yields from 1-adamantanecarbonitrile according to the following reaction scheme:

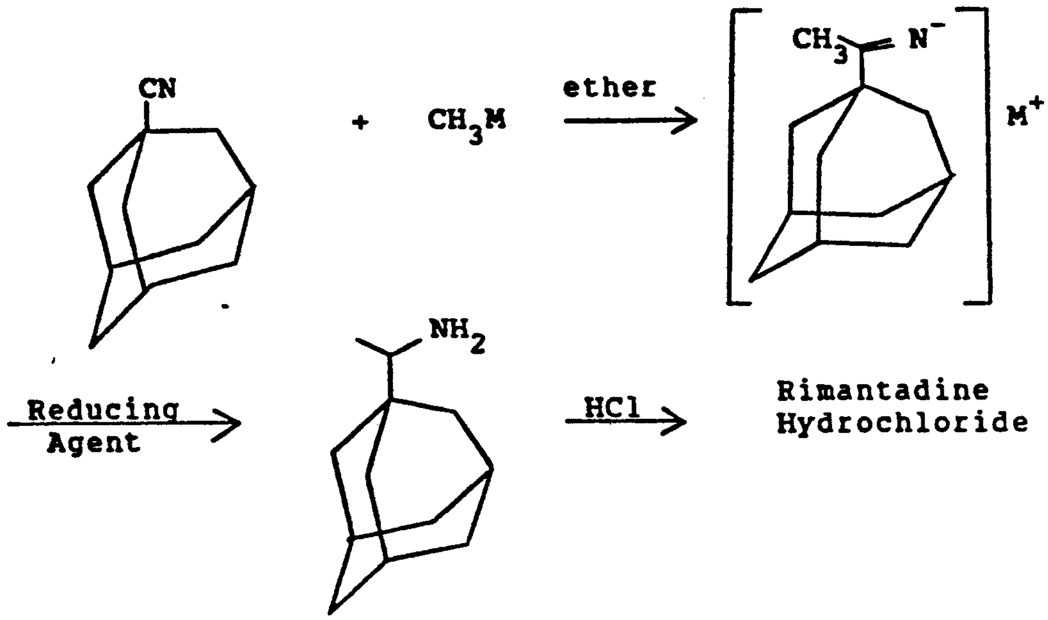


The starting carbonitrile is available commercially or can be prepared according to any procedure known in the art.

A key to the present process is the carbanion attack of the carbonitrile group using an organometallic reagent $CH_3M$ where M is lithium or MgX wherein X is Br or Cl. The negatively charged methyl moiety attacks the electropositive carbon center of the carbonitrile group to generate an intermediate imino compound. The organometallics are commercially available or the Grignard reagents may be generated <u>in situ</u> from a methyl halide and magnesium. Methyllithium is the preferred organometallic for the high yield and short reaction time possible, however, due to the

pyrophoric nature of methyllithium, methylmagnesium bromide is also preferred for its ease of handling.

Any liquid ether can be used as a solvent in which to carry out the organometallic reaction. Mixed ether/hydrocarbon solvent systems, such as tetrahydrofuran/toluene, can also be used as solvent for the organometallic reaction. Diethyl ether is preferred due to the generally excellent yields obtained; however, tetrahydrofuran is also preferred because of its higher boiling point. Other ethers that can be used include saturated aliphatic ethers such as methyl ether, methyl ethyl ether, ethyl propyl ether, i-propyl ether and n-propyl ether; cyclic ethers such as ethylene oxide, and the propylene oxides; diethers and triethers such as methylal, acetal, dioxan, and trioxymethylene; and aromatic ethers such as anisole, phenetole, phenyl ether, benzyl ether, and naphthyl ether. The organometallic reaction is conducted at a temperature in the range of -20°C to the reflux temperature of the solvent. A temperature of 0°C to 25°C is preferred.

The intermediate imino compound is reduced to rimantadine free base by any of a number of well-known reducing agents. Reduction with lithium aluminum hydride or with hydrogen in the presence of a platinum on carbon catalyst are preferred. Lithium aluminum hydride is particularly preferred, especially when the reduction is carried out in the same ether solvent as the initial step of the process. When lithium aluminum hydride is employed as the reducing agent, it can be added directly to the cooled reaction mixture from the organometallic reaction and the mixture is then heated to reflux. When catalytic hydrogenation is employed, the organometallic reaction is quenched by addition of a protic solvent, e.g. an alcohol such as ethanol, and

concentrated. The concentrate is diluted with a protic solvent, preferably the same protic solvent used in the quench, and a catalyst such as 5% Pt/C is added. The hydrogenation is conducted at a hydrogen pressure of 25 to 115 psia (170-790 kPa) at room temperature.

The preferred platinum on carbon catalyst used in the reduction step can be any of the many well-known such catalysts. While the particular composition of the catalyst is not believed to be critical, a particularly useful catalyst has been found to be 5% by weight platinum metal on carbon black particles sold by Johnson Matthey Inc. under the name Type 18MA. In general, such catalysts may contain about 2 to 20% by weight platinum on carbon based on the total weight of catalyst.

The invention can be further understood by reference to the following examples in which parts and percentages are by weight and temperature in degrees Centigrade.

Example 1

A small, round-bottomed reaction flask equipped with a mechanical stirrer, a condenser with a packed drying tube, an addition funnel and a thermometer was flushed with nitrogen. Into the flask was placed 6.4 g of 1-adamantanecarbonitrile and 120 ml anhydrous ethyl ether. The solution was stirred and cooled to 0-10°. An ethyl ether solution of methyl lithium 40 ml (1.55M) was added dropwise with stirring and cooling to maintain the temperature under 10°. After 30 minutes of stirring at this temperature, the cooling bath was removed and the reaction mixture was allowed to warm to room temperature over one hour.

Lithium aluminum hydride, 3.5 g, was added to the stirring mixture and the mixture was refluxed for approximately 2-1/2 hours, and allowed to cool to room temperature.

To the stirred mixture was added 4 ml of water, 12 ml of 15% sodium hydroxide and 4 ml of water consecutively. An exothermic reaction ensued and subsided after completion of the final water addition. The mixture was diluted with small amounts of ethyl ether and the layers were separated.

The ether layer was first dried with potassium hydroxide pellets and then anhydrous magnesium sulfate. The drying agents were each removed by filtration and HCl gas was bubbled into the clear ether solution of rimantadine free base. A white precipitate was collected and dried under vacuum to give 7.8 g of rimantadine hydrochloride in 90.7% yield.

## Example 2

To a small reaction flask equipped with a mechanical stirrer, a condenser with a packed drying tube, an addition funnel and a thermometer were placed 3.2 g of 1-adamantanecarbonitrile and 60 ml of anhydrous tetrahydrofuran (THF). Stirring was commenced and a clear solution resulted. The solution was cooled to 0-10° and 10.4 ml (2.9 molar ratio) of methyl magnesium bromide in THF was added dropwise at this temperature over 40 minutes. The cooling was removed and the mixture was allowed to warm to room temperature over ~3 hours. The mixture was again cooled to 5-10° and 1.75 g of lithium aluminum hydride was added. The mixture was then refluxed for two hours, and allowed to cool to room temperature.

Water, 2 ml, followed by 6 ml 15% NaOH solution was added. After adding another 2 ml of water, the

mixture was extracted with small portions of THF. The combined THF solution was dried with potassium hydroxide pellets and then magnesium sulfate. The drying agents each were removed and the clear solution of rimantadine free base was treated with hydrogen chloride gas to give a white precipitate. The solid was collected by filtration and dried in vacuum oven at 90° to give 3.6 g of rimantadine hydrochloride (83.7% yield).

Example 3

Ethyl ether, 60 ml, and 1-adamantanecarbonitrile, 3.2 g, were placed in a small reaction flask equipped with a stirrer, a condenser with a packed drying tube, an addition funnel and a thermometer. Stirring and cooling was initiated and a clear solution resulted. At 0-10°, 20 ml of 1.55 M methyl lithium was added dropwise over 45 minutes. The solution was allowed to warm to 15° over 20 minutes. Then, 50 ml of ethanol was added dropwise between 10-15°. An exothermic reaction took place with concommitant release of gas.

The ethanol solution was concentrated under vacuum to give a crude viscous oil. The oil was dissolved in 200 ml of ethanol and transferred to a Parr hydrogenation flask. Catalyst, 1.0 g 5% Pt/C was added to the flask and the flask purged with hydrogen. The hydrogenation was conducted at 30 psi (344 KPa) pressure and at room temperature overnight.

The catalyst was removed by filtration and washed with a small portion of ethyl ether. The combined organic layer was concentrated under vacuum and the resultant liquid was dissolved in ethyl ether. Hydrogen chloride gas was bubbled into the ether solution of rimantadine free base and a white solid

precipitated. The white solid was collected by filtration to give 2.4 g of rimantadine hydrochloride (56% yield).

Example 4

In a small, round-bottomed reaction flask equipped with a stirrer, a condenser with a packed drying tube, an addition funnel and a thermometer was placed 3.2 g of 1-adamantanecarbonitrile. Tetrahydrofuran (THF), 60 ml, was added and the mixture was stirred and cooled to 5-10°. Methyl magnesium chloride, 10.1 ml, (2.9 M solution THF) was added dropwise over 30 minutes.

The mixture was refluxed overnight and then cooled to 10°. To this cooled mixture, 1.75 g lithium aluminum hydride was added and the mixture refluxed for 2 hours.

The mixture was cooled with an ice bath and 2 ml of water followed by 6 ml 15% NaOH solution were added. An exothermic reaction ensued. The organic layer was dried first with potassium hydroxide pellets and then anhydrous magnesium sulfate. The drying agents were each removed by filtration and the clear solution of rimantadine free base was concentrated under vacuum. The oil was diluted with small portions of ethyl ether and hydrogen chloride gas was bubbled into this solution. A white precipitate formed and was isolated and dried to give 3.5 g rimantadine hydrochloride (81% yield).

WHAT IS CLAIMED IS: BP-6268

1. A process for the preparation of rimantadine comprising: contacting 1-adamantanecarbonitrile in an ether solvent or a mixed ether/hydrocarbon solvent system with $CH_3M$ where M is lithium or MgX wherein X is Br or Cl, and contacting the intermediate compound with a reducing agent to form rimantadine free base.

2. The process of Claim 1 wherein the rimantadine free base is contacted with hydrogen chloride to form rimantadine hydrochloride.

3. The process of Claim 1 wherein M is lithium or MgBr.

4. The process of Claim 3 wherein the reducing agent is hydrogen contacted with the intermediate compound in the presence of a platinum on carbon catalyst, or lithium aluminum hydride.

5. The process of Claim 4 wherein the ether solvent is ethyl ether or tetrahydrofuran.

6. A process for the preparation of rimantadine comprising: contacting 1-adamantanecarbonitrile in ethyl ether with methyl lithium, and contacting the resulting intermediate in ethyl ether with lithium aluminum hydride to form rimantadine free base.

7. The process of Claim 6 wherein rimantadine free base is recovered from the reaction mixture.

8. The process of Claim 6 wherein rimantadine free base is contacted with hydrogen chloride to form rimantadine hydrochloride.